# EUROPEAN PATENT SPECIFICATION

(11) **EP 1 542 679 B1**
(45) Date of publication and mention of the grant of the patent: **17.01.2007**
(21) Application number: 03795160.5
(22) Date of filing: 08.09.2003
(51) Int. Cl.: A61K 31/415, A61P 31/18, C07D 231/18, C07C 255/54

(54) **4-(3,5-DICYANOPHENOXY) PYRAZOLE DERIVATIVES FOR USE AS TRANSCRIPTASE MODULATORS IN THE TREATMENT OF I.A. HIV**
4-(3,5-DICYANOPHENOXY)PYRAZOL-DERIVATE ZUR VERWENDUNG ALS REVERSE TRANSKRIPTASE MODULATOREN ZUR BEHANDLUNG VON U.A. HIV
DERIVES DE 4-(3,5-DICYANOPHENOXY)PYRAZOLE UTILISES COMME MODULATEURS DE LA TRANSCRIPTASE INVERSE DANS LE TRAITEMENT, ENTRE AUTRES, DU VIH

(30) Priority: 16.09.2002 GB 0221477; 08.10.2002 GB 0223354
(43) Date of publication of application: 22.06.2005
(73) Proprietor: Pfizer Limited, Sandwich, Kent CT13 9NJ (GB); PFIZER INC., New York, N.Y. 10017 (US)
(72) Inventor: MOWBARY, C.E., c/oPfizer Global Res.& Development, Sandwich, Kent CT13 9NJ (GB); PRICE, D.A., c/oPfizer Global Res. & Development, Sandwich, Kent CT13 9NJ (GB); SELBY, M.D., c/oPfizer Global Res. & Development, Sandwich, Kent CT13 9NJ (GB); STUPPLE, P.A., c/oPfizer Global Res. & Development, Sandwich, Kent CT13 9NJ (GB)
(74) Representative: Swarbrick, Terry Mark
(86) International application number: PCT/IB2003/003946
(87) International publication number: WO 2004/024147

(56) References cited:
- WO-A-02/04424
- WO-A-02/085860
- US-A- 3 303 200

## Description

This invention relates to pyrazole derivatives, to their use in medicine, to compositions containing them, to processes for their preparation and to intermediates used in such processes.

Reverse transcriptase is implicated in the infectious lifecycle of Human Immunodeficiency Virus (HIV). Compounds which interfere with the function of this enzyme have shown utility in the treatment of conditions caused by HIV and genetically related retroviruses, such as Acquired Immune Deficiency Syndrome (AIDS). There is a constant need to provide new and better modulators, especially inhibitors, of HIV reverse transcriptase, since the virus is able to mutate, becoming resistant to the effects of known modulators.

A class of N-phenylpyrazoles which act as reverse transcriptase inhibitors are disclosed in *J. Med Chem.,* 2000, **43**, 1034 and in international application WO 02/04424. Antiviral activity is ascribed to a class of N-(hydroxyethyl)pyrazole derivatives in US patent number 3,303,200. International Application No. PCT/IB02/01234, unpublished at the filing date of the instant application, generically embraces, but does not specifically disclose, the compound of formula (I) below.

The compounds of the invention bind to the enzyme reverse transcriptase and are modulators, especially inhibitors, thereof.

According to the invention there is thus provided the compound of formula (I) 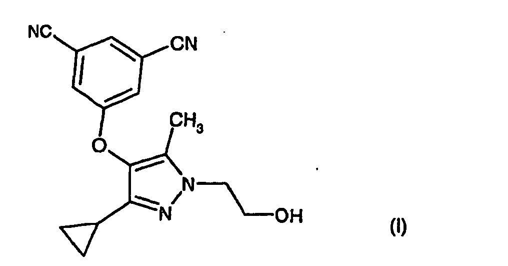 or a pharmaceutically acceptable salt or solvate thereof.

The pharmaceutically acceptable salts of the compounds of formula (I) include the acid addition and the base salts thereof.

Suitable acid addition salts are formed from acids which form non-toxic salts and examples are the hydrochloride, hydrobromide, hydroiodide, chloride, bromide, iodide, sulphate, bisulphate, nitrate, phosphate, hydrogen phosphate, acetate, fumarate, pamoate, aspartate, besylate, carbonate, bicarbonate/, camsylate, D and L-lactate, D and L-tartrate, esylate, mesylate, malonate, orotate, gluceptate, methylsulphate, stearate, glucuronate, 2-napsylate, tosylate, hibenzate, nicotinate, isethionate, malate, maleate, citrate, gluconate, succinate, saccharate, benzoate, esylate, and pamoate salts.

Suitable base salts are formed from bases which form non-toxic salts and examples are the sodium, potassium, aluminium, calcium, magnesium, zinc, choline, diolamine, olamine, arginine, glycine, tromethamine, benzathine, lysine, meglumine and diethylamine salts.

For reviews on suitable salts see Berge et al, J. Pharm. Sci., 66, 1-19, 1977 and Bighley *et al,* Encyclopedia of Pharmaceutical Technology, Marcel Dekker Inc, New York, 1996, Vol 13, pp453-497

The pharmaceutically acceptable solvates of the compounds of formula (I) include the hydrates thereof.

The compound of formula (I) may be modified to provide pharmaceutically acceptable derivatives thereof at any of the functional groups in the compound. Examples of such derivatives are described in: Drugs of Today, Volume 19, Number 9, 1983, pp 499 - 538; Topics in Chemistry, Chapter 31, pp 306 - 316; and In "Design of Prodrugs" by H. Bundgaard, Elsevier, 1985, Chapter 1 (the disclosures in which documents are incorporated herein by reference) and include: esters, carbonate esters, hemi-esters, phosphate esters, nitro esters, sulfate esters, sulfoxides, amides, sulphonamides, carbamates, azo-compounds; phosphamides, glycosides, ethers, acetals and ketals.

The invention encompasses all isomers of the compound of formula (I) and pharmaceutically acceptable salts or solvates thereof, including all geometric, tautomeric and optical forms, and mixtures thereof (e.g. racemic mixtures).

Separation of diastereoisomers may be achieved by conventional techniques, e.g. by fractional crystallisation, chromatography or high performance liquid chromatography (HPLC) of a stereoisomeric mixture of compounds. An individual enantiomer of a compound may also be prepared from a corresponding optically pure intermediate or by resolution, such as by HPLC of the corresponding racemate using a suitable chiral support, or by fractional crystallisation of the diastereoisomeric salts formed by reaction of the corresponding racemate with a suitable optically active acid or base, as appropriate.

The compound of formula (I) and pharmaceutically acceptable salts or solvates thereof may have the ability to crystallize in more than one form, a characteristic known as polymorphism, and all such polymorphic forms ("polymorphs") are encompassed within the scope of the invention. Polymorphism generally can occur as a response to changes in temperature or pressure or both, and can also result from variations in the crystallization process. Polymorphs can be distinguished by various physical characteristics, and typically the x-ray diffraction patterns, solubility behaviour, and melting point of the compound are used to distinguish polymorphs.

The compound of formula (I), pharmaceutically acceptable salts and solvates thereof, isomers thereof, and polymorphs thereof, are hereinafter referred to as the compounds of the invention.

Preferred compounds of the invention are the compound of formula (I) and its pharmaceutically acceptable salts and solvates thereof.

The most preferred compound of the invention is the compound of formula (I).

The compounds of the invention exhibit advantageous properties, including excellent metabolic stability, leading to excellent pharmacokinetic properties, in addition, the compounds of the invention may have advantages over those of the prior art with regard to a number of other useful properties, such as potency, duration of action, spectrum of activity, side effect profile, solubility, chemical stability, and so on.

The compounds of the invention may be prepared by any method known in the art for the preparation of compounds of analogous structure. The compounds of the invention can be prepared by the procedures described in the methods below, or by the specific methods described in the Examples, or by similar methods to either. The invention also encompasses any one or more of these processes for preparing the compounds of the invention.

The compound of formula (I) may be prepared according to the route shown in Scheme 1 below.

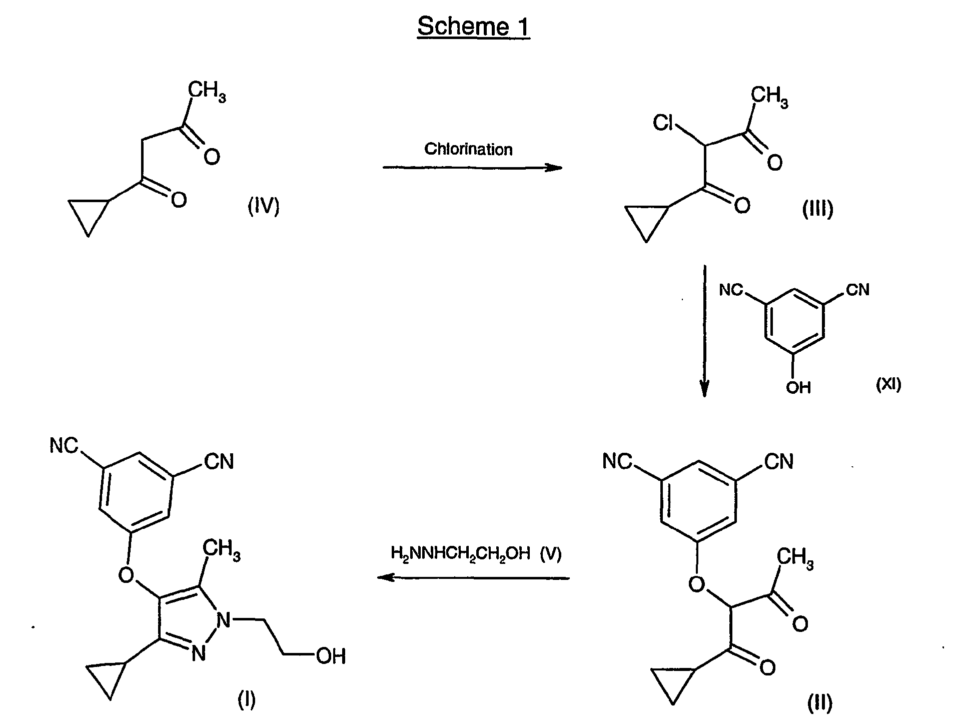

In Scheme 1, the compound of formula (I) may be prepared by condensation of the compound of formula (II) with 2-hydroxyethylhydrazine of formula (V) or a salt or hydrate thereof, optionally in the presence of an acid or a base, the base preferably being a tertiary amine base such as triethylamine and the acid preferably being acetic acid. In a typical procedure, a solution of the compound of formula (II) in a suitable solvent, such as acetic acid, is treated with the hydrazine of formula (V), or the salt or hydrate thereof, and, if used, the appropriate acid or base, at a temperature of from room temperature to the reflux temperature of the solvent. In a preferred procedure, the reaction is carried out at room temperature.

Functional equivalents of the compound of formula (II) may also be used in this reaction. These include compounds of formulae (VI) or (VII), in which L¹ and L², respectively, are each suitable leaving groups, preferably -N(C₁-C₆ alkyl)₂, most preferably -N(CH₃)₂.

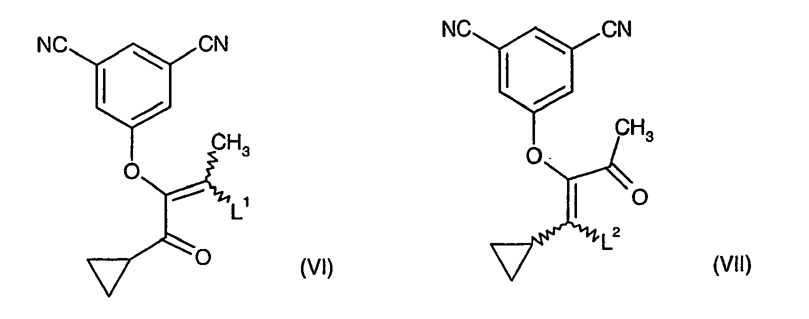

Thus, the compound of formula (I) may be prepared by condensation of a compound of formulae (VI) or (VII) with the compound of formula (V), or a salt or hydrate thereof, optionally in the presence of an acid or a base, the base preferably being a tertiary amine base such as triethylamine and the acid preferably being acetic acid. In a typical procedure, a solution of the compound of formula (VI) or (VII) in a suitable solvent, such as ethanol, is treated with the compound of formula (V), or the salt or hydrate thereof, and, if used, the appropriate acid or base, at a temperature of from room temperature to the reflux temperature of the solvent. In a preferred procedure, the reaction mixture is heated under reflux.

Compounds of formula (VI) in which L¹ is dimethylamino may be prepared by the reaction of the compound of formula (VIII) with an appropriately substituted formamide acetal at an elevated temperature, preferably at about 100°C. Compounds of formula (VII) in which L¹ is dimethylamino may be prepared by the reaction of the compound of formula (IX) under the same conditions.

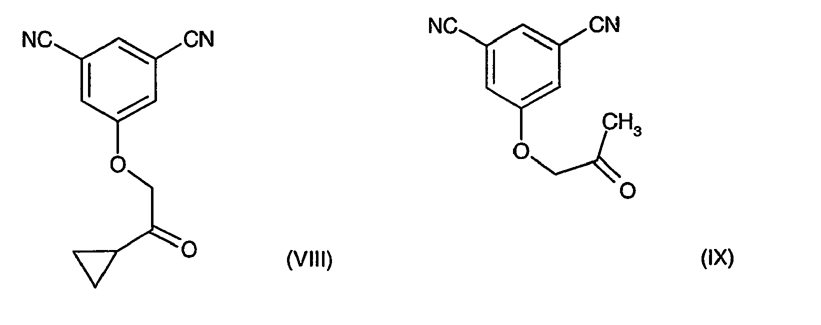

The compound of formula (VIII) is either commercially available or may be prepared by the reaction of the compound of formula (X) 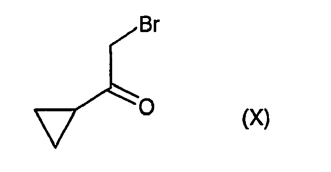 with the compound of formula (XI)

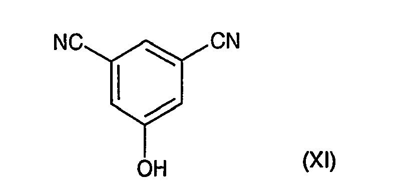

In a typical procedure, a solution of the compound of formula (XI) in a suitable solvent, such as acetone, is treated with a suitable base, such as caesium carbonate, and the compound of formula (X). In a preferred procedure, the reaction mixture is heated, for example under reflux. Optionally, a nucleophilic catalyst such as sodium iodide or tetrabutylammonium iodide may be added.

The compound of formula (IX) is either commercially available or may be prepared from the compound of formula (XII) 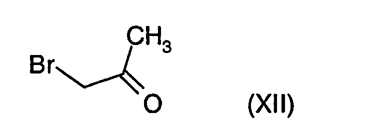 and the compound of formula (XI) in the same way that the compound of formula (VIII) may be prepared from the compound of formula (X).

The compound of formula (II) may be prepared by the reaction of the compound of formula (III) with the compound of formula (XI).

In a typical procedure, a solution of the compound of formula (III) in a suitable solvent such as acetone is treated with the compound of formula (XI) and a suitable base, such as potassium or caesium carbonate, and heated, preferably under reflux. Optionally, a nucleophilic catalyst such as sodium iodide or tetrabutylammonium iodide may be added.

The compound of formula (III) is either commercially available or may be prepared by reaction of the compound of formula (IV) with a chlorinating reagent. In a typical procedure, a cooled solution of the compound of formula (IV) in a suitable solvent such as acetonitrile is treated first with tetrabutylammonium bromide and chlorotrimethylsilane and then dry dimethylsulphoxide. In another typical procedure, the compound of formula (IV) is treated with sulphuryl chloride, optionally in the presence of a suitable solvent such as dichloromethane.

The compound of formula (I) may also be prepared by reaction of the pyrazole of formula (XIII) 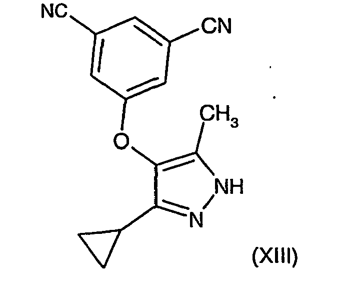 with an alkylating agent of formula (XIV)

Lg-CH₂CH₂OH (XIV)

or a protected derivative thereof.

In a typical procedure, a solution of the pyrazole of formula (XIII) in a suitable solvent such as ethanol or N,N-dimethylformamide is treated with an alkylating agent of formula (XIV) such as a protected hydroxyethyl bromide and a base such as sodium ethoxide or sodium hydride and heated at a temperature of from 0°C to the reflux temperature of the solvent. A preferred combination is N,N-dimethylformamide as the solvent, sodium hydride as the base, 0°C as the temperature and 2-(2-bromoethoxy)tetrahydro-2*H*-pyran as the alkylating agent.

As will be appreciated by the skilled artisan, in the alkylation of the pyrazole of formula (XIII) it may be necessary or desirable to protect the OH group of the compound of formula (XIV), in which case the compound of formula (I) is finally prepared by deprotection of the corresponding compound bearing an -OP¹ group, wherein P¹ is a suitable protecting group. Examples of suitable protecting groups will be apparent to the skilled person; see, for instance, 'Protecting groups in Organic Synthesis (Second Edition)' by Theodora W. Green and Peter G. M. Wuts, 1991, John Wiley and Sons (in particular pages 10 - 118, relating to protection for the hydroxyl group), incorporated herein by reference. Such compounds bearing an -OP¹ group may be prepared using the routes described above, *mutatis mutandis.*

Compounds of formulae (IV) and (V) are either commercially available, known from the literature or easily prepared by methods well known to those skilled in the art.

The compounds of the invention can be administered alone, but will generally be administered in admixture with a suitable pharmaceutical excipient, diluent or carrier selected with regard to the intended route of administration and standard pharmaceutical practice.

For example, the compounds of the invention can be administered orally, buccally or sublingually in the form of tablets, capsules, multi-particulates, gels, films, ovules, elixirs, solutions or suspensions, which may contain flavouring or colouring agents, for immediate-, delayed-, modified-, sustained-, pulsed- or controlled-release applications. The compounds of the invention may also be administered as fast-dispersing or fast-dissolving dosage forms or in the form of a high energy dispersion or as coated particles. Suitable formulations of the compounds of the invention may be in coated or uncoated form, as desired.

Such solid pharmaceutical compositions, for example, tablets, may contain excipients such as microcrystalline cellulose, lactose, sodium citrate, calcium carbonate, dibasic calcium phosphate, glycine and starch (preferably corn, potato or tapioca starch), disintegrants such as sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included.

### General Example

A formulation of the tablet could typically contain from 0.01 mg to 500mg of active compound whilst tablet fill weights may range from 50mg to 1000mg. An example of a formulation for a 10mg tablet is illustrated below:

| ingredient | %w/w |
|---|---|
| Compound of the invention | 10.000* |
| Lactose | 64.125 |
| Starch | 21.375 |
| Croscarmellose sodium | 3.000 |
| Magnesium Stearate | 1.500 |

| | |
|---|---|
| * Quantity adjusted in accordance with drug activity. | |

The tablets are manufactured by a standard process, for example, direct compression or a wet or dry granulation process. The tablet cores may be coated with appropriate overcoats.

Solid compositions of a similar type may also be employed as fillers in gelatin or HPMC capsules. Preferred excipients in this regard include lactose, starch, a cellulose, milk sugar or high molecular weight polyethylene glycols. For aqueous suspensions and/or elixirs, the compounds of the invention may be combined with various sweetening or flavouring agents, colouring matter or dyes, with emulsifying and/or suspending agents and with diluents such as water, ethanol, propylene glycol and glycerin, and combinations thereof.

The compounds of the invention can also be administered parenterally, for example, intravenously, intra-arterially, intraperitoneally, intrathecally, intraventricularly, intraurethrally, intrasternally, intracranially, intramuscularly or subcutaneously, or they may be administered by infusion or needleless injection techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

For oral and parenteral administration to human patients, the daily dosage level of the compounds of the invention will usually be from 0.01 to 30 mg/kg, preferably from 0.01 to 5 mg/kg (in single or divided doses).

Thus tablets or capsules of the compound of the invention may contain from 1 to 500 mg of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention. The skilled person will appreciate that, in the treatment of certain conditions the compounds of the invention may be taken as a single dose as needed or desired.

The compounds of invention can also be administered intranasally or by inhalation and are conveniently delivered in the form of a dry powder inhaler or an aerosol spray presentation from a pressurised container, pump, spray, atomiser or nebuliser, with or without the use of a suitable propellant, e.g. dichlorodifluoromethane, trichlorofluoromethane, dichlorotetrafluoroethane, a hydrofluoroalkane such as 1,1,1,2-tetrafluoroethane (HFA 134A [trade mark]) or 1,1,1,2,3,3,3-heptafluoropropane (HFA 227EA [trade mark]), carbon dioxide or other suitable gas. In the case of a pressurised aerosol, the dosage unit may be determined by providing a valve to deliver a metered amount. The pressurised container, pump, spray, atomiser or nebuliser may contain a solution or suspension of the active compound, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of a compound of the invention and a suitable powder base such as lactose or starch.

Alternatively, the compounds of the invention can be administered in the form of a suppository or pessary, or they may be applied topically in the form of a gel, hydrogel, lotion, solution, cream, ointment or dusting powder. The compounds of the invention may also be dermally or transdermally administered, for example, by the use of a skin patch. They may also be administered by the pulmonary or rectal routes.

They may also be administered by the ocular route. For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

For application topically to the skin, the compounds of the invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compounds of the invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

It is to be appreciated that all references herein to treatment include curative, palliative and prophylactic treatment.

Oral administration is preferred.

Included within the scope of the invention are embodiments comprising compositions containing a compound of the invention along with one or more additional therapeutic agents. Such a composition is especially useful for the prevention and/or treatment of infection by HIV and related retroviruses which may evolve rapidly into strains resistant to any monotherapy. Alternatively, additional therapeutic agents may be desirable to treat diseases and conditions which result from or accompany the disease being treated with the compound of the invention. For example, in the treatment of an HIV or related retroviral infection, it may be desirable to additionally treat opportunistic infections, neoplasms and other conditions which occur as a result of the immuno-compromised state of the patient being treated.

Preferred combinations of the invention include compositions comprising a compound of the invention and one or more:
(a) reverse transcriptase inhibitors such as abacavir, adefovir, didanosine, lamivudine, stavudine, zalcitabine and zidovudine;
(b) non-nucleoside reverse transcriptase inhibitors such as capavirine, delavirdine, efavirenz, and nevirapine;
(c) HIV protease inhibitors such as indinivir, nelfinavir, ritonavir, and saquinavir;
(d) CCR5 antagonists such as TAK-779 or UK-427,857;
(e) CXCR4 antagonists such as AMD-31 00;
(f) integrase inhibitors, such as L-870,810 or S-1360;
(g) inhibitors of viral fusion such as T-20;
(h) investigational drugs such as trizivir, KNI-272, amprenavir, GW-33908, FTC, PMPA, MKC-442, MSC-204, MSH-372, DMP450, PNU-140690, ABT-378, KNI-764, DPC-083, TMC-120 or TMC-1 25;
(i) antifungal agents, such as fluconazole, itraconazole or voriconazole; or
(j) antibacterial agents, such as azithromycin.

The activity of the compounds of the invention as reverse transcriptase inhibitors may be measured using the following assay.

### Inhibition of HIV-1 reverse transcriptase enzyme

Using purified recombinant HIV-1 reverse transcriptase (RT, EC, 2.7.7.49) obtained by expression in Escherichia Coli, a 96-well plate assay system is established for assaying a large number of samples using either the Poly(rA)-oligo(dT) Reverse Transcriptase [3H]-SPA enzyme assay system (Amersham NK9020) or the [3H]-flashplate enzyme assay system (NEN - SMP 103) and following the manufacturer's recommendations. The compounds are dissolved in 100% DMSO and diluted with the appropriate buffer to a 5% final DMSO concentration. The inhibitory activity is expressed in percent inhibition relative to DMSO control. The concentration at which compound inhibits reverse transcriptase by 50% is expressed as the IC₅₀ of the compound.

The compound of Example 1, when tested according to the above procedure, had an IC₅₀ value of 295 nanomolar.

The metabolism of the compounds of the invention may be measured using the following assays.

### A. Metabolism in human liver microsomes and Supermix™

The metabolic vulnerability of the compounds of the invention in microsomes and Supermix™ may be assayed as follows.

The microsomal fraction is isolated from several human livers and the P450 content determined. Supermix™ is obtained from Gentest. Human microsomes (0.5µM cytochrome P450) and Supermix™ (0.05µM cytochrome P450) are added to an incubation media containing 50mM phosphate buffer (pH7.4), 5mM MgCl₂, 1 mM NADP and an NADPH generating system consisting of isocitrate and isocitrate dehydrogenase. The substrate concentration is 1µM and incubations are carried out at 37°C for 1 hour. Samples are taken at time points throughout this period and analysed using hplc-ms-ms assay.

The compound of Example 1, when tested according to the above procedure, had a t ½ value of >120 minutes (both human microsomal and Supermix ™).

### B. Metabolism in Human hepatocytes.

The metabolic vulnerability of the compounds of the invention in human hepatocytes may be assayed as follows.

Cryopreserved human hepatocytes are obtained from *In vitro* Technologies, Inc. The hepatocytes are thawed and suspended at 1 million cells/ml in 50% Krebs-Heinsleit buffer : 50% Williams E media containing 10% foetal bovine serum. The substrate concentration is 3µM and incubations are carried out at 37°C for 3 hours. Samples are taken at time points throughout this period and analysed using hplc-ms-ms assay.

The compound of Example 1, when tested according to the above procedure, had an unbound hepatocyte clearance value of <9 ml/min/kg.

Thus the invention provides:
(i) the compound of formula (I) or a pharmaceutically acceptable salt or solvate thereof;
(ii) a process for the preparation of the compound of formula (I) or a pharmaceutically acceptable salt or solvate thereof;
(iii) a pharmaceutical composition including the compound of formula (I) or a pharmaceutically acceptable salt or solvate thereof, together with a pharmaceutically acceptable excipient, diluent or carrier;
(iv) the compound of formula (I) or a pharmaceutically acceptable salt, solvate or composition thereof, for use as a medicament; (v) the compound of formula (I) or a pharmaceutically acceptable salt, solvate or composition thereof, for use as a reverse transcriptase inhibitor or modulator;
(vi) the compound of formula (I) or a pharmaceutically acceptable salt, solvate or composition thereof, for use in the treatment of an HIV or genetically-related retroviral infection, or a resulting acquired immune deficiency syndrome (AIDS);
(vii) the use of the compound of formula (I) or of a pharmaceutically acceptable salt, solvate or composition thereof, for the manufacture of a medicament having reverse transcriptase inhibitory or modulating activity;
(viii) the use of the compound of formula (I) or of a pharmaceutically acceptable salt, solvate or composition thereof, for the manufacture of a medicament for the treatment of an HIV or genetically-related retroviral infection, or a resulting acquired immune deficiency syndrome (AIDS);

The following Examples illustrate the preparation of the compounds of formula (I). The synthesis of certain intermediates used therein are described in the Preparations section that follows the Examples.

¹H Nuclear magnetic resonance (NMR) spectra were in all cases consistent with the proposed structures. Characteristic chemical shifts (δ) are given in parts-per-million (ppm) downfield from tetramethylsilane using conventional abbreviations for designation of major peaks, e.g.: s, singlet; d, doublet; t, triplet; q, quartet; m, multiplet; br, broad. The following abbreviations have been used: HRMS, high resolution mass spectrometry; hplc, high performance liquid chromatography; nOe, nuclear Overhauser effect; m.p., melting point; CDCl₃, deuterochloroform; D₆-DMSO, deuterodimethylsulphoxide; CD₃OD, deuteromethanol. Where thin layer chromatography (TLC) has been used it refers to silica gel TLC using silica gel 60 F₂₅₄ plates, R_{f} being the distance travelled by a compound divided by the distance travelled by the solvent front on the TLC plate.

### EXAMPLE 1

### 5-{[3-Cyclopropyl-1-(2-hydroxyethyl)-5-methyl-1H-pyrazol-4-yl]oxy}isophthalonitrile

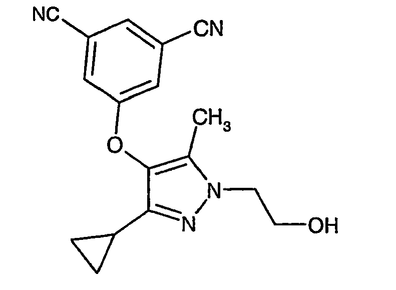

2-Hydroxyethylhydrazine (1.15ml, 16.9mmol) was added to a solution of the diketone from Preparation 7 (4.1 g, 15.4mmol) in acetic acid (40ml) under nitrogen at room temperature. After stirring for 18 hours, the mixture was concentrated under reduced pressure and the residual oil was purified by flash chromatography on silica gel eluting with ethyl acetate:pentane (50:50 changing to 100:0, by volume) to provide samples of the two regioisomers which required further purification.

The less polar fraction was isolated as a yellow solid (1.2g), a sample of which (815mg) was purified by recrystallisation from toluene (5ml) to give the title compound as colourless needles (600mg). A sample of this material (580mg) was further purified by preparative HPLC using a Luna C8(II) 150x21.2mm 10µm column eluting with 95:5 water:acetontrile (0.1 % aqueous trifluoroacetic acid) and acetonitrile (0-1 min 100:0 then over 1 min changing to 70:30 for 18min then changing to 100:0 over 1 min) to provide a sample of the title compound. This material was freed of any remaining acid by dissolving in dichloromethane (50ml) and washing with saturated aqueous sodium bicarbonate solution (50ml). The organic phase was dried over magnesium sulphate, filtered and concentrated under reduced pressure to provide a foam (270mg) which was recrystallised from toluene (5ml) to give a sample of the title compound as colourless needles (265mg). M.p. 127-128 °C.
¹H NMR (400MHz, CDCl₃): δ = 0.84 (m, 4H), 1.58 (m, 1H), 2.13 (s, 3H), 4.03 (m, 2H), 4.13 (m, 2H), 7.42 (s, 2H), 7.59 (s, 1H).
LRMS (atmospheric pressure chemical ionisation) : m/z [MH⁺] 309.
Microanalysis: Found C, 66.14; H, 5.24; N, 18.15. C₁₇H₁₆N₄O₂ requires C, 66.22; H, 5.23; N, 18.17%.
Regioisomer confirmed by nOE NMR and unambiguously identified by X-ray crystallography.

The more polar fraction was further purified by flash chromatography on silica gel eluting with ethyl acetate:toluene (50:50, by volume) to give 5-{[5-cyclopropyl-1-(2-hydroxyethyl)-3-methyl-1*H*-pyrazol-4-yl]oxy}isophthalonitrile (structure below) 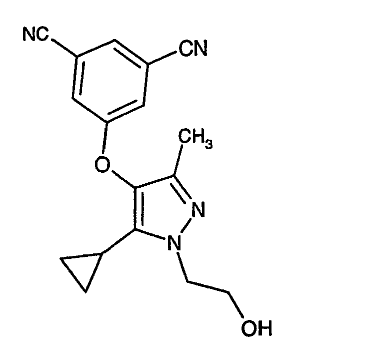 as a white solid (90mg). M.p. 129-130 °C.
¹H NMR (400MHz, CDCl₃): δ = 0.68 (m, 2H), 0.87 (m, 2H), 1.58 (m, 1H), 2.03 (s, 3H), 4.07 (m, 2H), 4.31 (m, 2H), 7.39 (s, 2H), 7.59 (s, 1H).
LRMS (atmospheric pressure chemical ionisation): m/z [MH⁺] 309.

### EXAMPLE 2

### 5-{[3-Cyclopropyl-1-(2-hydroxyethyl)-5-methyl-1H-pyrazol-4-yl]oxy}isophthalonitrile

To a stirred solution of the pyrazole from Preparation 9 (250mg, 0.64mmol) in methanol (6ml) was added *para*-toluenesulfonic acid (12mg, 0.06mmol). After 18 hours the reaction mixture was concentrated and the residue was partitioned between 10% aqueous potassium carbonate solution (20ml, w/v) and dichloromethane (20ml). The separated aqueous layer was washed with dichloromethane (2 x 20ml) and the combined organic components were dried over magnesium sulfate, filtered and concentrated to give the title compound as a pale yellow oil (195mg) which was used without further purification.
¹H NMR (400mHz, CDCl₃) consistent with that described above.
LRMS (thermospray): m/z [MH⁺]309.

### PREPARATION 1

### 1,3-Dibromo-5-methoxybenzene

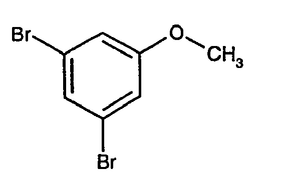

Sodium methoxide (8.80ml of a 4.5M solution in methanol, 39.6mmol) was added dropwise to a stirred solution of 3,5-dibromofluorobenzene (5.00g, 19.0mmol) (Aldrich) in *N,N*-dimethylformamide (95ml) at 0°C under nitrogen. The reaction was allowed to warm to room temperature, stirred for 1 hour and then concentrated under reduced pressure. The residue was dissolved in ether (500ml) and the resulting solution was washed with water (3x300ml) and brine (300ml), dried over magnesium sulphate, filtered and concentrated under reduced pressure to provide the title compound (5.13g) as a white solid.
¹H-NMR (300MHz, CDCl₃): δ = 3.79 (s, 3H), 7.00 (s, 2H), 7.26 (s, 1H).
LRMS (thermospray): m/z [MH⁺] 266.
Microanalysis: Found: C, 31.56; H, 2.29. C₇H₆OBr₂ requires C, 31.62; H, 2.27%.

### PREPARATION 2

### 3,5-Dicyanomethoxybenzene

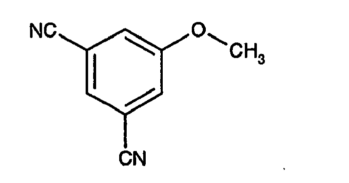

Tris(dibenzylideneacetone)dipalladium(0) (6.53g, 7.15mmol) was added in one portion to a stirred solution of the bromide of Preparation 1 (38.0g, 143mmol), 1,1'-bis(diphenylphosphino)ferrocene (9.3g, 16.8mmol) and zinc cyanide (20.0g, 172mmol) in *N,N*-dimethylformamide (300ml) at room temperature under nitrogen. The reaction was heated at 100°C for 14 hours and cooled to room temperature. Water (1500ml) was added and the mixture was extracted with ethyl acetate (3x500ml). The combined organics were filtered and the filtrate was washed with water (500ml), dried over magnesium sulphate, filtered and concentrated under reduced pressure. The resulting solid was triturated with toluene (1000ml) to provide the title compound (18.0g) as a tan solid.
¹H-NMR (300MHz, CDCl₃): δ = 3.83 (3H, s), 7.31 (2H, s), 7.48 (1 H, s).

### PREPARATION 3

### 3.5-Dicyanohyrdroxybenzene

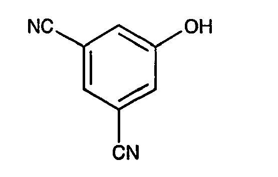

The nitrile of Preparation 2 (9.60g, 60.7mmol) was added portionwise to a stirred suspension of aluminium trichloride (32.4g, 243mmol) in dichloromethane (250ml) at 0°C under nitrogen. The suspension was heated to 45°C and stirred for 6 days. The reaction was cooled to room temperature and cautiously poured onto ice (450ml). Concentrated hydrochloric acid (450ml) was added dropwise and the resulting suspension was stirred for 10 minutes at room temperature. The resulting solid was collected by filtration, washed with water and dried over phosphorus pentoxide to provide the title compound (7.83g) as a tan solid containing approximately 11 % starting material by ¹H-NMR and microanalysis.
¹H-NMR (400MHz, CDCl₃): δ = 7.36 (m, 2H), 7.56 (m, 1H).

### PREPARATION 4

### 3-Oxobutanoic acid

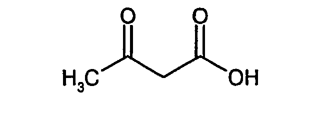

Sodium hydroxide (37.9g, 0.947mol) was dissolved in water (770ml) and was added dropwise over 20min to 3-oxo-butanoic acid methyl ester (1 00g, 0.861 mol) (Aldrich) at room temperature with stirring. The reaction was stirred for 18 hours, quenched with ammonium sulfate (700g) and acidified slowly with a solution of concentrated hydrochloric acid (21.5ml) in water (250ml) with ice cooling. The reaction mixture was extracted with diethylether (6x200ml) and the combined organic extracts were dried over magnesium sulphate, filtered and concentrated under reduced pressure to provide the title compound (58.2g) as a pale yellow oil which was a mixture of keto:enol tautomers (as observed in ¹H NMR).
¹H NMR (400MHz, CDCl₃): δ = 2.00 (s, 3H-enol), 2.30 (s, 3H-keto), 3.51 (s, 2H-keto), 5.02 (s, 1H-enol).

### PREPARATION 5

### 1-Cyclopropyl-1,3-butanedione

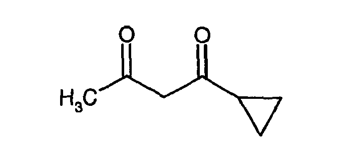

Magnesium turnings (3.04g, 125mmol) suspended in methanol (145ml) were heated to reflux under nitrogen for 1 hour, cooled to room temperature and the β-keto acid from Preparation 4 (25.5g, 250mmol) dissolved in methanol (25ml) was added dropwise with ice-cooling. The reaction was stirred for 1 hour at room temperature and the solvent was removed under reduced pressure to give the magnesium salt of the acid. Meanwhile, cyclopropane-carboxylic acid (9.91 ml, 125mmol) was dissolved in dimethylformamide (200ml) and carbonyldiimidazole (22.4g, 138mmol) was added portionwise under nitrogen at 0°C. This was stirred for 1.5 hour and the magnesium salt from above was added as a solution in dimethylformamide (100ml) at 0°C. The reaction was allowed to stir at room temperature for 92 hours and the mixture was poured into 2M aqueous hydrochloric acid (85ml) then diluted with water (170ml). The mixture was extracted with diethylether (6x200ml) and the combined organic extracts were washed with brine (3x200ml), dried over magnesium sulphate and concentrated under reduced pressure. The residual orange oil was purified by flash chromatography on silica gel eluting with pentane:diethylether (100:0 changing to 90:10 then 80:20, by volume) to provide the title compound (7.39g) as a yellow oil.
¹H NMR (400MHz, CDCl₃): δ = 0.89 (m, 2H), 1.08 (m, 2H), 1.59 (m, 1H), 2.00 (s, 3H), 5.61 (s, 1H), 15.62 (s, 1H).
LRMS (electrospray) : m/z [MNa⁺] 149.

### PREPARATION 6

### 2-Chloro-1-cyclopropyl-1,3-butanedione

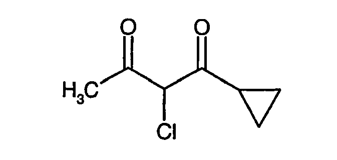

Chlorotrimethylsilane (18.9ml, 174mmol) was added to a solution of tert-butylammonium bromide (932mg, 2.89mmol) in dry acetonitrile (50ml) under nitrogen at room temperature and the mixture was cooled to 0°C. The diketone from Preparation 5 (7.3g, 57.9mmol) in acetonitrile (36ml) was then added followed by dropwise addition of dry dimethylsulfoxide (12.3ml, 174mmol). The reaction was stirred at 0°C for 1.5 hours and the mixture was diluted with water (500ml), extracted with diethylether (2x200ml and 100ml) and the combined organic extracts were dried over magnesium sulphate, filtered and concentrated under reduced pressure. The residual oil was purified by flash chromatography on silica gel eluting with pentane:diethylether (100:0 changing to 95:5 then 90:10, by volume) to provide the title compound (5.76g) as a colourless oil.
¹H NMR (400MHz, CDCl₃): δ = 1.04 (m, 2H), 1.18 (m, 2H), 2.27 (s, 3H), 2.42 (m, 1H), 15.78 (s, 1H).
LRMS (electrospray): m/z [M-H⁺] 159.

### PREPARATION 7

### 5-[1-(Cyclopropylcarbonyl)-2-oxopropoxy]isophthalonitrile

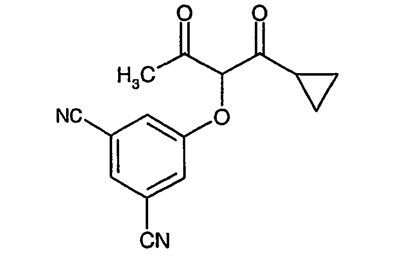

Cesium carbonate (6.01 g, 18.5mmol) and the phenol from Preparation 3 (2.66g, 18.5mmol) were added to a stirred solution of the diketone from Preparation 6 (2.46g, 15.4mmol) in acetone (75ml) under nitrogen at 60°C and the reaction was stirred for 3 hours. After cooling the acetone was removed under reduced pressure and the residue was partitioned between 1N aqueous hydrochloric acid (100ml) and dichloromethane (100ml). The aqueous phase was separated and extracted with dichloromethane (50ml). The combined organic components were dried over magnesium sulphate and concentrated under reduced pressure to give the title compound as a cream solid (4.2g).
¹H NMR (400MHz, CDCl₃): δ = 0.92 (m, 2H), 1.19 (m, 2H), 1.78 (m, 1H), 1.99 (s, 3H), 7.47 (m, 2H), 7.62 (m, 1H).
LRMS (electrospray) : m/z [M-H⁺] 267.

### PREPARATION 8

### 5-[(3-Cyclopropyl-5-methyl-1H-pyrazol-4-yl)oxy]isophthalonitrite

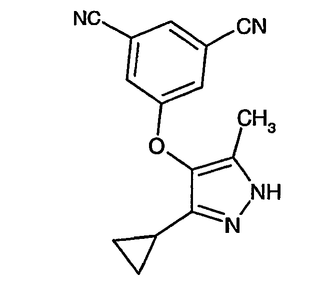

Hydrazine hydrate (298µl, 6.16mmol) was added to a solution of the diketone from Preparation 7 (1.50g, 5.60mmol) in acetic acid (22ml) under nitrogen at room temperature. After stirring at 50 °C for 18 hours, the mixture was allowed to cool to room temperature and concentrated under reduced pressure. The residue was partitioned between 10% aqueous potassium carbonate solution (50ml) and dichloromethane (50ml). The separated aqueous layer was washed twice with dichloromethane (2x50ml). The combined organic components were dried over magnesium sulphate, filtered and concentrated under reduced pressure to provide a pale yellow oil. The crude product mixture was purified by flash chromatography on silica gel eluting with pentane:ethyl acetate (75:25 changing to 67:33, by volume) to provide the title compound (1.20g) as a pale yellow oil.
¹H NMR (400MHz, CDCl₃): δ = 0.75 (m, 2H), 0.85 (m, 2H), 1.60 (m, 1H), 2.10 (s, 3H), 7.40 (s, 2H), 7.60 (s, 1H).
LRMS (thermospray): m/z [MH⁺] 260.

### PREPARATION 9

### 5-({3-Cyclopropyl-5-methyl-1-[2-(tetrahydro-2H-pyran-2-yloxy)ethyl]-1H-pyrazol-4-yl}oxy)isophthalonitrile

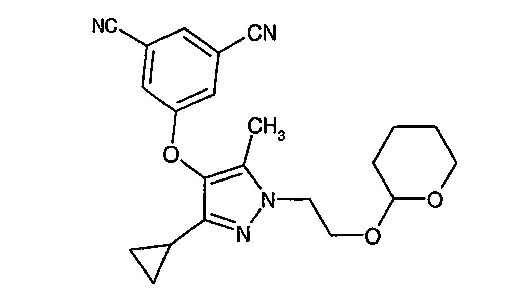

To a stirred solution of the pyrazole from Preparation 8 (420mg, 1.59mmol) in dimethylformamide (4ml) at 0°C was added sodium hydride (70mg of a 60% w/w suspension in mineral oil). After the addition was complete the cooling bath was removed and the mixture was stirred at room temperature for 30 minutes. A solution of 2-(2-bromoethoxy)tetrahydro-2H-*pyran* (264µl, 1.75mmol) in dimethylformamide (2ml) was added. After 2 hours the reaction mixture was quenched by addition of water (20ml) and was extracted with dichloromethane (3 x 20ml). The combined organic components were washed with brine (2 x 20ml), dried over magnesium sulfate, filtered and concentrated to give a yellow oil. The crude product mixture was purified by flash chromatography on silica gel eluting with dichloromethane:methanol (100:0 changing to 98:2, by volume) to provide a mixture of the two regioisomers (594mg). The two regioisomers were separated by flash chromatography on silica gel eluting with toluene:ethyl acetate (100:0 changing to 80:20, 75:25, 67:33 and 50:50 by volume) to provide the title compound (257mg) (less polar fraction) and its regioisomer (90mg) (more polar fraction).
Less Polar Fraction
¹H NMR (400MHz, CDCl₃): δ = 0.78 (m, 4H), 1.55 (m, 5H), 1.67 (m, 2H), 2.12 (s, 3H), 3.45 (m, 1H), 3.65 (m, 1H), 3.75 (m, 1H), 4.04 (m, 1 H), 4.18 (m, 2H), 4.53 (m, 1H), 7.40 (s, 2H), 7.59 (s, 1H).
LRMS (thermospray): m/z [MH⁺] 393.

### More Polar Fraction

### 5-({5-Cyclopropyl-3-methyl-1-[2-(tetrahydro-2H-pyran-2-yloxy)ethyl]-1H-pyrazol-4-yl}oxy)isophthalonitrile

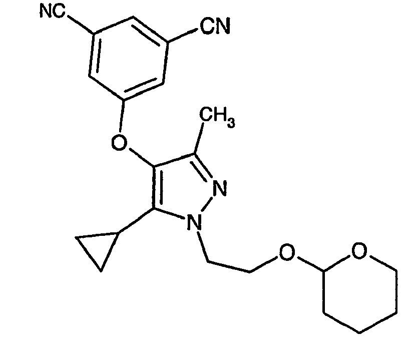

¹H NMR (300MHz, CDCl₃): δ = 0.68 (m, 2H), 0.85 (m, 2H), 1.53 (m, 3H), 1.72 (m, 4H), 2.10 (s, 3H), 3.51 (m, 1H), 3.72 (m, 1H), 3.83 (m, 1H), 4.17 (m, 1H), 4.35 (m, 2H), 4.58 (m, 1H), 7.38 (s, 2H), 7.59 (s, 1H).
LRMS (thermospray): m/z [MH⁺] 393.

## Claims

1. The compound of formula (I) 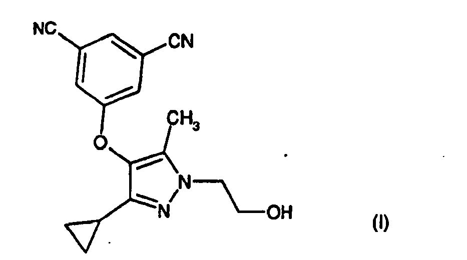 or a pharmaceutically acceptable salt or solvate thereof.

2. A pharmaceutical composition including the compound of formula (I) according to claim 1 or a pharmaceutically acceptable salt or solvate thereof together with one or more pharmaceutically acceptable excipients; diluents or carriers.

3. A pharmaceutical composition according to claim 2 including one or more additional therapeutic agents.

4. The compound of formula (I) according to claim 1 or a pharmaceutically acceptable salt or solvate thereof, or a pharmaceutical composition according to claim 2 or 3, for use as a medicament.

5. The compound of formula (I) according to claim 1 or a pharmaceutically acceptable salt or solvate thereof, or a pharmaceutical composition according to claim 2 or 3, for: use as a reverse transcriptase inhibitor or modulator.

6. The compound of formula (I) according to claim 1 or a pharmaceutically acceptable salt or solvate or thereof, or a pharmaceutical composition according to claim 2 or 3, for use In the treatment of an HIV or genetically-related retroviral infection, or a resulting acquired immune deficiency syndrome (AIDS).

7. Use of the compound of formula (I) according to claim 1 or a pharmaceutically acceptable salt or solvate thereof, or a pharmaceutical composition according to claim 2 or 3, for the manufacture of a medicament having reverse transcriptase inhibitory or modulating activity.

8. Use of the compound of formula (I) according to claim 1 or a pharmaceutically acceptable salt or solvate thereof, or a pharmaceutical composition according to claim 2 or 3, for the manufacture of a medicament for the treatment of an HIV or genetically-related retroviral infection, or a resulting acquired immune deficiency syndrome (AIDS).

9. A process for preparing the compound of formula (I) according to claim 1 or a salt or solvate thereof, which comprises:
(A) condensation of a compound of formulae (II), (VI) or (VII) 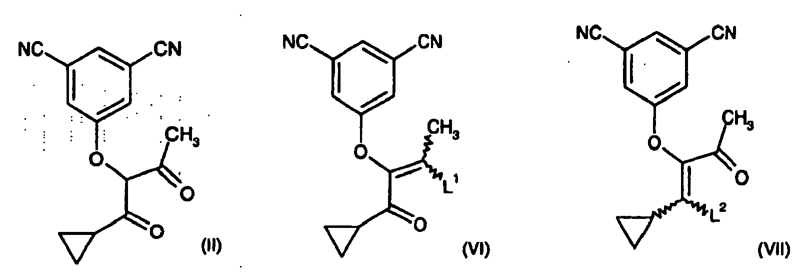 wherein L¹ and L² are suitable leaving groups with the compound of formula (V)
H₂NNHCH₂CH₂OH (V)
or a salt or hydrate thereof;
(B) alkylation of the pyrazole of formula (XIII) 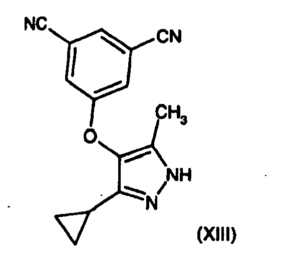 with an alkylating agent of formula (XIV)
Lg-CH₂CH₂OH (XIV)
or a protected derivative thereof; wherein Lg is a suitable leaving group;
(C) deprotecting a protected derivative of the compound of formula (I);
and optionally converting the compound of formula (I) prepared by any one of processes (A) to (C) into pharmaceutically acceptable salt or solvate thereof.

## Patentansprüche

1. Verbindung der Formel (I) 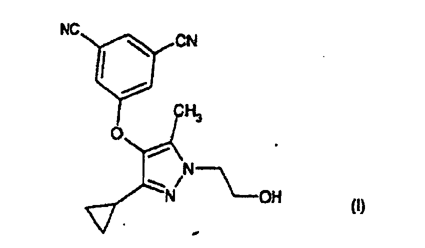 oder ein pharmazeutisch akzeptables Salz oder Solvat derselben.

2. Pharmazeutische Zusammensetzung, die die Verbindung der Formel (I) nach Anspruch 1 oder ein pharmazeutisch akzeptables Salz oder Solvat derselben zusammen mit einem oder mehreren pharmazeutisch akzeptablen Streckmitteln, Verdünnungsmitteln oder Trägern umfasst.

3. Pharmazeutische Zusammensetzung nach Anspruch 2, die ein oder mehrere zusätzliche therapeutische Mittel umfasst.

4. Verbindung der Formel (I) nach Anspruch 1 oder ein pharmazeutisch akzeptables Salz oder Solvat derselben oder eine pharmazeutische Zusammensetzung nach Anspruch 2 oder 3 zur Verwendung als Medikament.

5. Verbindung der Formel (I) nach Anspruch 1 oder ein pharmazeutisch akzeptables Salz oder Solvat derselben oder eine pharmazeutische Zusammensetzung nach Anspruch 2 oder 3 zur Verwendung als Reverse-Transkriptase-Inhibitor oder -Modulator.

6. Verbindung der Formel (I) nach Anspruch 1 oder ein pharmazeutisch akzeptables Salz oder Solvat derselben oder eine pharmazeutische Zusammensetzung nach Anspruch 2 oder 3 zur Verwendung bei der Behandlung einer HIV- oder genetisch bedingten Retrovirusinfektion oder eines infolgedessen erworbenen Immunschwächesyndroms (AIDS).

7. Verwendung der Verbindung der Formel (I) nach Anspruch 1 oder eines pharmazeutisch akzeptablen Salzes oder Solvats derselben oder einer pharmazeutischen Zusammensetzung nach Anspruch 2 oder 3 zur Herstellung eines Medikaments, das reverse Transkriptase hemmende oder modulierende Aktivität aufweist.

8. Verwendung der Verbindung der Formel (I) nach Anspruch 1 oder eines pharmazeutisch akzeptablen Salzes oder Solvats derselben oder einer pharmazeutischen Zusammensetzung nach Anspruch 2 oder 3 zur Herstellung eines Medikaments zur Behandlung einer HIV- oder genetisch bedingten Retrovirusinfektion oder eines infolgedessen erworbenen Immunschwächesyndroms (AIDS).

9. Verfahren zur Herstellung der Verbindung der Formel (I) nach Anspruch 1 oder eines Salzes oder Solvats derselben, das umfasst:
(A) Kondensation einer Verbindung der Formeln (II), (VI) oder (VII) 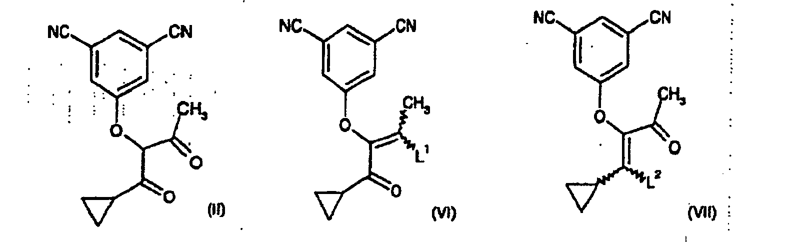 worin L¹ und L² geeignete Abgangsgruppen sind, mit der der Verbindung der Formel (V)
H₂NNHCH₂CH₂OH (V)
oder einem Salz oder Hydrat derselben;
(B) Alkylierung des Pyrazols der Formel (XIII) 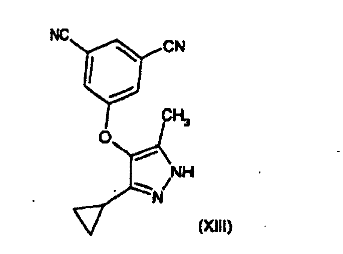 mit einem Alkylierungsmittel der Formel (XIV)
Lg-CH₂CH₂OH (XIV)
oder einem geschützten Derivat desselben, worin Lg eine geeignete Abgangsgruppe ist;
(C) Entschützen eines geschützten Derivats der Verbindung der Formel (I);
und optionales Umwandeln der durch eines der Verfahren (A) bis (C) hergestellten Verbindung der Formel (I) in ein pharmazeutisch akzeptables Salz oder Solvat derselben.

## Revendications

1. Composé de formule (I) 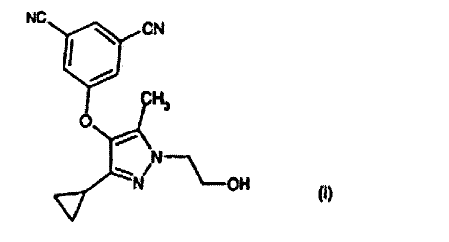 ou un de ses sels ou produits de solvatation pharmaceutiquement acceptables.

2. Composition pharmaceutique comprenant le composé de formule (I) suivant la revendication 1, ou un de ses sels ou produits de solvatation pharmaceutiquement acceptables, conjointement avec un ou plusieurs excipients, diluants ou supports pharmaceutiquement acceptables.

3. Composition pharmaceutique suivant la revendication 2, comprenant un ou plusieurs agents thérapeutiques supplémentaires.

4. Composé de formule (I) suivant la revendication 1, ou un de ses sels ou produits de solvatation pharmaceutiquement acceptables, ou composition pharmaceutique suivant la revendication 2 ou 3, pour une utilisation comme médicament.

5. Composé de formule (I) suivant la revendication 1, ou un de ses sels ou produits de solvatation pharmaceutiquement acceptables, ou composition pharmaceutique suivant la revendication 2 ou 3, pour une utilisation comme inhibiteur ou modulateur de la transcriptase inverse.

6. Composé de formule (I) suivant la revendication 1, ou un de ses sels ou produits de solvatation pharmaceutiquement acceptables, ou composition pharmaceutique suivant la revendication 2 ou 3, pour une utilisation dans le traitement d'une infection par le VIH ou un rétrovirus génétiquement apparenté, ou d'un syndrome d'immunodéficience acquise (SIDA) qui en résulte.

7. Utilisation du composé de formule (I) suivant la revendication 1, ou d'un de ses sels ou produits de solvatation pharmaceutiquement acceptables, ou d'une composition pharmaceutique suivant la revendication 2 ou 3, pour la production d'un médicament ayant une activité d'inhibition ou de modulation de la transcriptase inverse.

8. Utilisation du composé de formule (I) suivant la revendication 1, ou d'un de ses sels ou produits de solvatation pharmaceutiquement acceptables, ou d'une composition pharmaceutique suivant la revendication 2 ou 3, pour la production d'un médicament destiné au traitement d'une infection par le VIH ou un rétrovirus génétiquement apparenté, ou d'un syndrome d'immunodéficience acquise (SIDA) qui en résulte.

9. Procédé pour la préparation du composé de formule (I) suivant la revendication 1, ou d'un de ses sels ou produits de solvatation, qui comprend :
(A) la condensation d'un composé de formule (II), (VI) ou (VII) 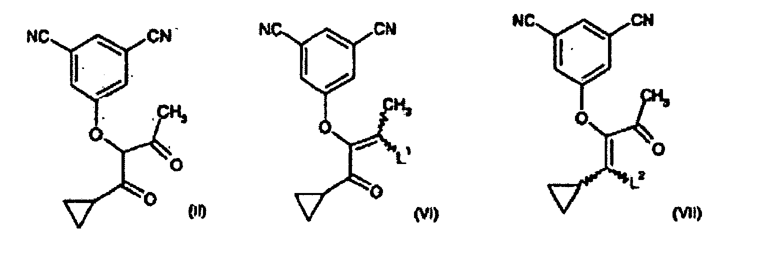 dans lesquelles L¹ et L² représentent des groupes partants convenables,
avec le composé de formule (V)
H₂NNHCH₂CH₂OH (V)
ou un de ses sels ou hydrates ;
(B) l'alkylation du pyrazole de formule (XIII) 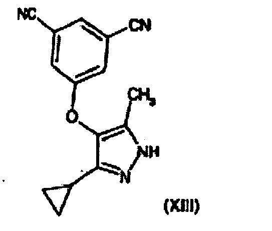 avec un agent alkylant de formule (XIV)
Lg-CH₂CH₂OH (XIV)
ou un de ses dérivés protégés, formule dans laquelle Lg représente un groupe partant convenable ;
(C) la suppression de la protection d'un dérivé protégé du composé de formule (I) ;
et facultativement la conversion du composé de formule (I) préparé par n'importe lequel des procédés (A) à (C) en un de ses sels ou produits de solvatation pharmaceutiquement acceptables.
